# EUROPEAN PATENT APPLICATION

(11) **EP 1 785 131 A1**
(43) Date of publication of application: **16.05.2007**
(21) Application number: 05781929.4
(22) Date of filing: 31.08.2005
(51) Int. Cl.: A61K 9/10, A61K 31/196, A61K 31/405, A61K 45/00, A61K 47/14, A61K 47/26, A61K 47/28, A61K 47/32, A61K 47/34, A61K 47/36, A61K 47/38, A61K 47/42, A61P 29/00

(54) **EXTERNAL PREPARATION OF S/O TYPE**

(30) Priority: 31.08.2004 JP 2004252829
(71) Applicant: ASPION CO., LTD., Tokushima-shi, Tokushima 7700815 (JP)
(72) Inventor: FUJII, Takeru, Tokushima 7720051 (JP); HIRATA, Akihiko, Tokushima-shi, Tokushima 770-0872 (JP); GOTO, Masahiro, Fukuoka 8190044 (JP); KAMIYA, Noriho, Fukuoka 8190002 (JP)
(74) Representative: Gille Hrabal Struck Neidlein Prop Roos
(86) International application number: PCT/JP2005/016355
(87) International publication number: WO 2006/025583

(57) **Abstract**

The present invention provides an external preparation which can improve a skin permeability of a hydrophilic medicine such as NSAID so that the medicine can act directly on a diseased area without passing through gastrointestinal tract or mucosa. The S/O type external preparation external preparation excellent in percutaneous absorbability of the present invention comprises a medicine-containing complex dissolved or dispersed in an oil phase, wherein the complex contains a hydrophilic medicine covered with a surfactant and is in a form of a solid.

## Description

### TECHNICAL FIELD

The present invention relates to an S/O (Solid-in-Oil) type external preparation which is excellent in percutaneous absorbability.

### BACKGROUND ART

Non-steroidal anti-inflammatory drugs (hereinafter referred to as "NSAID") are widely used because of their excellent anti-inflammatory and analgesic effects. However, the drugs are well known for causing gastric mucosa dysfunctions including gastrointestinal tract disturbance such as particularly gastric ulcer, gastric perforation and the like.

For example, aspirin which is a typical NSAID has been used for more than 100 years since it was discovered, but it is said that 50, 000 to 100, 000 and people are hospitalized and more than 2000 people die for gastrointestinal dysfunction annually in the United States. The drug disaster by side effects of NSAID other than aspirin is added, it is estimated that nearly 20, 000 people die annually.

Conventionally, the NSAID preparation has mainly been given as an oral preparation. However, an oral preparation reaches the maximum drug concentration in blood (Cmax) immediately after dosage, and soon after that, the drug concentration in blood drops sharply. As a result, it follows that side effects are tend to take place while the advantageous effects do not last long. Thus, in order to control Cmax and to maintain the advantageous effects at the same time, some preparations are taken three times a day. However, there is a problem that a desired medicinal effect is difficult to be obtained if the patient fails to follow the prescribed drug regimen.

On the other hand, an external preparation of NSAID has an advantage of acting directly in a diseased area, and additionally, of lower rate of gastrointestinal dysfunction. However, a skin permeability of NSAID is extremely low. It is probably because an NSAID generally has a carboxyl group as a substituent and high hydrophilicity, so an NSAID cannot penetrate a skin which consists of stratum corneum and the like. As a result, the problem arises that even if an external preparation of NSAID is taken, the NSAID cannot be delivered to a diseased area, thus sufficient effects cannot be obtained.

As for the other medicines too, it is difficult to use them for external preparation because of the high hydrophilicity, so some of them are developed mainly as oral preparations. As described above, if the hydrophilic medicines can be used for external preparations, not only the side effects can be inhibited but also durability of the medicinal effects can be enhanced.

The inventors of the present invention have developed an S/O/W type pharmaceutical preparation which is prepared by dispersing a lyophiled mixture of an aqueous solution of enzyme, bioactive peptide or medicine and an organic solvent solution of surfactant in an oil phase (S/O type pharmaceutical preparation, Solid in Oil), and dispersing the S/O type pharmaceutical preparation in an aqueous phase (Japanese Unexamined Patent Publication No.2004-43355; S.Okazaki, N.Kamiya, K.Abe, M.Goto, F.Nakashio, Biotechnol.Bioeng., 55(2), pp.455-460 (1997); N.Kamiya, S.Okazaki, M.Goto, Biotechnol.Tech., 11(6), pp.375-378 (1997)). For example, in Example of the Publication No.2004-43355, an S/O/W type pharmaceutical preparation containing insulin or irinotecan hydrochloride as a medicine is prepared. The experimental data showing suppression of leakage to an aqueous phase is also described.

In addition to the above, an enzyme composition covered with surfactant, which includes a surfactant, an enzyme, water and a salts, and has high activity even in an water-insoluble organic solvent is described in Japanese Unexamined Patent Publication No.6-303973. In PCT International Application Japanese Translation No.2003-501404, a pharmaceutical preparation in which solid phase particles consisting of dehydrated products of a medicine, a surfactant and a membrane permeation promoter are suspended in a delivery medium is described. The pharmaceutical preparation allows a therapeutic protein or peptide to be absorbed transmucosally in the oral cavity such as cheek and tongue or nose. Furthermore, a method for producing a hydrophobic preparation, in which a hydrophobic solvent is supplied around a hydrophilic substance covered with an amphiphilic substance is described in PCT International Application Japanese Translation No.10-510256.

### DISCLOSURE OF THE INVENTION

As described above, if a medicine which has been developed mainly as an oral preparation can be used for an external preparation, such an external preparation is considered to be of vital use. However, since a hydrophilic medicine has very low skin permeability, the medicine cannot achieve its sufficient medicinal effect when used for an external preparation. In spite of that, there is an example wherein a hydrophilic medicine was blended in an external preparation. However, since skin permeability of a hydrophilic medicine is improved by adding an organic solvent such as isopropanol in the pharmaceutical preparation, irritation and the like caused by the organic solvent may occur.

Also, an S/O type pharmaceutical preparation wherein a solid phase containing a hydrophilic medicine is dispersed in an oil phase has been conventionally known. However, such pharmaceutical preparations are aimed at oral administration or mucosal administration.

Under such a situation, it is an object of the present invention to provide a safe external preparation which is capable of improving skin permeability of hydrophilic medicines such as NSAID, maintaining its blood concentration without going through a gastrointestinal tract or mucosa, or acting directly in the diseased area.

In order to solve the above problems, inventors of the present invention have diligently studied about constituting components of an external preparation. As a result, the present invention has been accomplished with a surprising discovery that an S/O type pharmaceutical preparation which inventors of the present invention have already developed also improves the skin permeability of a hydrophilic medicine.

An S/O type external preparation of the present invention is characterized in that a medicine-containing complex which is excellent in percutaneous absorbability is dissolved or dispersed in an oil phase, wherein the complex contains a hydrophilic medicine covered with a surfactant and is in a form of a solid.

A method for improving percutaneous absorbability of a hydrophilic medicine is characterized in that: the hydrophilic medicine is covered with a surfactant so as to prepare a medicine-containing complex; and the complex is solved or dispersed in an oil phase for making an external preparation.

### BRIEF DESCRIPTION OF THE DRAWINGS

Fig.1 shows the variation of the concentration of diclofenac sodium in blood plasma with time when a diclofenac sodium-S/O type suspension of the present invention is applied to a rabbit's ear and when a diclofenac sodium suspension is administered orally to a rabbit.
Fig.2 shows the variation of concentration of diclofenac sodium in blood plasma with time when a diclofenac sodium-S/O type suspension of the present invention is applied to a dog's ear and when a conventional diclofenac sodium external preparation is applied.
Fig.3 shows a photograph (A) of a rabbit's ear to which a diclofenac sodium-S/O type suspension of the present invention has been applied, and a photograph (B) of a rabbit's ear to which a conventional diclofenac sodium external preparation has been applied. As shown in the Figures, a scar tissue and redness are seen when a conventional external preparation is applied, while no abnormality can be seen when a pharmaceutical preparation of the present invention is applied.

### BEST MODE FOR CARRYING-OUT OF THE INVENTION

An S/O type external preparation of the present invention is characterized in that a medicine-containing complex which is excellent in percutaneous absorbability is dissolved or dispersed in an oil phase, wherein the complex contains a hydrophilic medicine covered with a surfactant and is in a form of a solid.

First, a medicine-containing complex constituting an S/O type external preparation of the present invention will be explained.

A medicine-containing complex comprises a hydrophilic medicine and a surfactant as main constituting components, wherein the hydrophilic part of the surfactant associates the medicine and the surfactant envelopes the medicine. The complex may be composed only of the hydrophilic medicine and the surfactant in order to prevent the particle from becoming too large, or the complex may contain a pharmaceutical preparation component which is allowed to be contained in a pharmaceutical preparation.

The reason for using the hydrophilic medicine is that an object of the present invention is to improve percutaneous absorbability of the hydrophilic medicine with extremely low percutaneous absorbability compared with a hydrophobic medicine. Such hydrophilic medicines are not particularly limited, and examples include NSAIDs such as diclofenac sodium and indomethacin; antihypertensive medicines such as angiotensin-converting enzyme inhibitor, β blocking agent, calcium antagonist, and angiotensin II receptor blocker; antiemetics such as 5-HT3 antagonist; antianxiety medicine; antiepileptic medicine; stimulant; antiparkinsonian medicine; psychoneurotic agent; local anesthetic; muscle relaxant suxametonium; autonomic drug; antispasm drug; cardiac stimulant; antiarrhythmic medicine; diuretic agent; antihypertensive medicine; vasodilator; antihyperlipidemic medicine; antitussive agent; expectorant medicine; bronchodilator; constipating agent; peptic ulcer medicine; anabolic steroid; hormonal agents such as adrenal hormonal agent, androgenic agent, estrogen, and progestational agent; urinary organ agent; uterotonic; medicine for liver disease; antidote; antipodagric; enzyme preparation; antidiabetic medicine; antimetabolite; antihistamine; antileprosy medicine; synthetic antimicrobial; antiviral medicine; antiprotozoal agent; sulfa medicine; migraine medicine; antibiotic; hemostat; and anticoagulant. In addition, though it has conventionally been thought that protein cannot penetrate a dermal tissue, there is a possibility that protein can be used as an external preparation by the present invention. Proteins which are possibly used include cell growth factors such as fibroblast growth factor (FGF) and hepatocyte growth factor (HGF) ; antagonist of cell growth factor such as NK4 which is antagonist of HGF. Additionally, it is considered that a vaccine such as peptide vaccine and nucleic acid vaccine, and nucleic acid and the like may be used as an external preparation by the present invention. However, the lower the molecular weight of the medicine is, the higher its skin permeability is, so the molecular weight of hydrophilic medicine of the present invention is preferably 10,000 or less, more preferably 5,000 or less, even more preferably 1,000 or less.

Diclofenac sodium is especially exemplified as the hydrophilic medicine. The diclofenac sodium is commercially available as an external preparation, but its skin permeability is not sufficient yet. Additionally, the external preparation causes irritation due to an organic solvent such as isopropanol. However, the following examples have demonstrated that the present invention is capable of improving the skin permeability of diclofenac sodium.

The surfactant to be used in the present invention is not particularly limited insofar as it is pharmaceutically acceptable, and the examples include a nonionic surfactant, an anionic surfactant, a cationic surfactant, an amphoteric surfactant, and a bile salt.

Examples of the nonionic surfactant include polyglycerin condensed ricinolein acid ester, decaglycerin ester, glycerin fatty acid ester, polyglycerin fatty acid ester, polyoxy ethylene glycerin fatty acid ester, sorbitan fatty acid ester, polyoxyethylene sorbit fatty acid ester, polyoxy ethylene castor oil, hydrogenated castor oil, and sucrose fatty acid ester (sucrose stearic acid ester, sucrose palmitic acid ester, sucrose myristic acid ester, sucrose oleic acid ester, sucrose lauric acid ester, sucrose erucic acid ester, and sucrose mixed fatty acid ester). One of these may be selected or more than one may be selected and combined for use.

As the nonionic surfactant, an ester compound produced by using unsaturated fatty acid such as erucic acid or oleic acid as an ingredient is preferred, and more preferred are sucrose erucic acid ester, sucrose oleic acid ester, sucrose mixed fatty acid ester. Alternatively, one, or two or more selected from the group consisting of glycerin fatty acid ester, polyglycerin fatty acid ester, polyoxyethylene glycerin fatty acid ester, sorbitan fatty acid ester, sucrose fatty acid ester, polyoxy ethylene sorbitan fatty acid ester, polyoxy ethylene castor oil, and hydrogenated castor oil may be used.

As the surfactant, a surfactant of high hydrophobicity having a HLB value of 10 or less is preferably used, because such a surfactant becomes easy to dissolute or disperse a medicine-containing complex in an oil phase.

To the medicine-containing complex of the present invention, a stabilizing agent may be added. As the stabilizing agent used in the present invention, a hydrophilic protein or polysaccharide which has a molecular weight of 10,000 or more is preferably used. The stabilizing agent is capable of improving the stability of the complex and inhibiting the leakage of the medicine to the outside the complex in external preparation by being covered by the surfactant together with the medicine and the like.

Examples of the protein as the stabilizing agent include serum albumin (molecular weight: about 67,000), ovalbumin (molecular weight: about 45,000), casein (molecular weight: about 19, 000 or more), lysozyme (molecular weight: about 14, 000 or more), and lipase (molecular weight: about 45, 000) . One of these may be selected or more than one may be selected and combined for use. Preferably, one or more than one selected from a group consisting of serum albumin, ovalbumin, and casein may be used.

Examples of the polysaccharide as the stabilizing agent include LM pectin, HM pectin, hydroxypropylmethyl cellulose phthalate, heparin, alginic acid, and carboxymethyl cellulose, and one of these may be selected or more than one may be selected and combined for use. Preferably, one or more than one selected from a group consisting of LM pectin, HM pectin and hydroxypropylmethyl cellulose phthalate may be used.

It is to be noted that the molecular weight of the protein or the polysaccharide varies depending on its origin and the like. For example, while pectin generally has a molecular weight ranging from 50,000 to 150,000, the pectin used in the example described later has a molecular weight ranging from 20,000 to 40,000. However, the molecular weight of the components exemplified in the above is generally 10, 000 or more. Herein, as for the molecular weight, catalogue values of the protein and the polysaccharide to be used may be referred to, but if the molecular weight is unknown, the molecular weight can be measured as the number average molecular weight and the like.

In the medicine-containing complex, the mass ratio of the stabilizing agent with respect to the hydrophilic medicine is preferably in the range of 0.01 to 100. If it is less than 0.01, its effect may not be sufficiently achieved, while if it is more than 100, the amount of medicine occupied in the complex becomes small so that the original effects of the medicine may not be exerted. More preferably, it is within the range of 0.1 to 10, even more preferably 0.5 to 5.

In case that the stabilizing agent is blended, a weight ratio of the surfactant with respect to the combined amount of the hydrophilic medicine and the stabilizing agent is preferably within the range of 0.5 to 100, more preferably 1 to 50, even more preferably 2 to 25.

The S/O type external preparation of the present invention contains a solution or a suspension wherein the medicine-containing complex is dissolved or dispersed in an oil phase. Whether the preparation becomes a solution or a suspension depends on the type and amount of surfactant and oil phase, and with or without treatment of sonication and the like.

The oil phase used in the present invention is not particularly limited insofar as it is pharmaceutically acceptable, and examples include vegetable oil, animal oil, neutral lipid such as mono-substituted, di-substituted, or tri-substituted glyceride, synthetic oil, and sterol derivatives.

Specific examples include vegetable oils such as soybean oil, cottonseed oil, rape seed oil, sesame oil, corn oil, peanut oil, safflower oil, sunflower oil, olive oil, rape seed oil, perilla oil, fennel oil, cacao seed oil, cinnamon oil, mentha oil, and bergamot oil; animal oils such as beef fat, pork oil, and fish oil; neutral lipids such as medium-chain fatty acid triglyceride, triolein, trilinolein, tripalmitin, tristearin, trimyristin, and triarachidonin; synthetic lipids such as azone; sterol derivatives such as cholesteryloleate, cholesteryl linoleate, cholesteryl myristate, cholesteryl palmitate, and cholesteryl arachidate; long chain fatty acid esters such as isopropyl myristate, octyldodecyl myristate, cetyl myristate, oleic acid ethyl, ethyl linoleate, isopropyl linoleate, isopropyl palmitate, and butyl stearate; lactate esters such as ethyl lactate and cetyl lactate; a multivalent carboxylic acid ester such as triethyl citrate, diisopropyl adipate, diethyl sebacate, and diisopropyl sebacate; other carboxylic acid esters such as cetyl 2-ethylhexanoate; hydrocarbons such as petrolatum and paraffin squalane; and silicones. One of these may be selected or more than one may be selected and combined for use. Preferably, one or more than one selected from a group consisting of soybean oil, sesame oil, olive oil, safflower oil, sunflower oil, canola oil, and perilla oil is used. Particularly it is preferred to use triglyceride or cooking oils based on the same, and practically preferred is soybean oil, especially highly-purified soybean oil. In addition, preferably a neutral lipid or long chain fatty acid ester, more preferably a long chain fatty acid ester, even more preferably an isopropyl myristate may be used.

The ratio of the oil phase contained in solution or suspension contained in the S/O type external preparation of the present invention differs depending on the type of oil component and other constituting components and the like, and is preferably within the range of 50 to 99.5 w/v %, more preferably 60 to 90 w/v%.

The solution or suspension of the medicine-containing complex can be prepared by the process comprising the steps of:
(1) mixing a hydrophilic medicine with a organic solvent solution containing a surfactant and if necessary a stabilizing agent, to prepare a W/O type emulsion;
(2) drying the W/O type emulsion to prepare a medicine-containing complex; and
(3) dissolving or dispersing the medicine-containing complex in an oil phase.

### (1) Step of preparing a W/O type emulsion

In the step, first, an aqueous solution of the hydrophilic medicine is prepared. If necessary, the stabilizing agent is also added. The water used is exemplified by, for example, pure water, purified water, distilled water, saline, buffer. A small amount of water miscible organic solvent such as ethanol may be added as necessary. However, caution should be taken as it becomes difficult to form an emulsion if too much alcohol and the like are added.

The concentration of the hydrophilic medicine and the stabilizing agent in the aqueous solution is not particularly limited as long as they can be substantively completely dissolved, and for example, the concentration may be about 5 to 30 mg/mL.

Separately, an organic solvent solution of the surfactant is prepared. The organic solvent is not particularly limited insofar as it is able to dissolve the surfactant and removable by diffusion in the subsequent step. The examples of such solvent include aliphatic hydrocarbons such as hexane; and aromatic hydrocarbons such as toluene. The concentration is not particularly limited, and may be, for example, about 1 to 10 % by mass.

Next, a W/O type emulsion is prepared by mixing the aqueous solution of the stabilizing agent and the hydrophilic medicine, and the organic solvent solution of the surfactant according to a conventional method. For example, high speed stirring using homogenizer, stirring by a stirrer such as propeller mixer or disper, and additionally irradiation of ultrasonic wave may be employed.

### (2) Drying step

In the step, the W/O type emulsion obtained in step (1) described above is dried to obtain the medicine-containing complex. The method of drying is not particularly limited. A method such as lyophilization and drying under reduced pressure condition may be employed, and lyophilization is preferable. As to specific conditions, a conventional method is employed. In this step, it is preferred to remove the water and the organic solvent substantially completely. Moisture may cause a medicine leakage in the pharmaceutical preparation, and the organic solvent may affect adversely on a living subject. To be more specific, the moisture content should be about 1% or less measured by the Karl Fischer method.

### (3) Dispersing step

In this step, the medicine-containing complex obtained in the step (2) described above is dissolved or dispersed in the oil phase, to thereby produce the S/O type solution or suspension. More specifically, as is the case in the step (1), high speed stirring using homogenizer, stirring by a stirrer such as propeller mixer or disper, and additionally irradiation of ultrasonic wave may be employed.

The amount of the oil phase used in this step is, for example, about 1 to 10 mL per 1g of the medicine-containing complex, although it depends on the kind and affinity of the surfactant and the oil phase, or the like.

The obtained S/O type pharmaceutical preparation may further be dispersed in an aqueous phase according to a conventional method to give an S/O/W type pharmaceutical preparation.

The above mentioned solution or suspension may be directly applied to diseased area, preferably another additive component is added to make it a pharmaceutical preparation. Examples of another additive component include diluent (for example, sugars such as sucrose; a starch derivative such as dextrin; cellulose derivatives such as armellose sodium; a water-soluble polymer such as xanthan gum), colorant, lubricant (for example, metal stearate such as calcium stearate and magnesium stearate; a laurylsulfuric acid salt such as laurylsulfuric acid sodium salt and laurylsulfuric acid magnesium; starch derivatives in said diluent and the like), bonding agent (such as foregoing diluent and macrogol), emulsifying agent, thickener, moisturizer (such as glycerin), stabilizer (for example, a p-hydroxybenzoic acid ester such as methyl paraben and propylparaben; an alcohol such as chlorobutanol, benzylalcohol, and phenylethylalcohol; benzalkonium chloride; a phenol such as phenol and cresol; thimerosal; acetic anhydride; sorbic acid, and the like), preservative, solvent (such as water, ethanol, and glycerin), solubilizing agent, suspending agent (such as carmellose sodium), buffer agent, pH adjuster, base (such as polyethylene glycol, crotamiton, diethyl sebacate, and petrolatum), and these are blended in a usual amount to be blended.

The above mentioned solution or suspension may be blended with the other additive component by a conventional method according to the form of each drug formulation, to obtain an ointment, a lotion agent, an aerosol agent, a plaster, and aqueous cataplasms. The present invention is not limited to these.

The dosage of the external preparation of the present invention can be adjusted according to the type and the amount of the medicine to be blended, and the age and the symptom of the patient, and the like.

A method for improving percutaneous absorbability of a hydrophilic medicine is characterized in that: the hydrophilic medicine is covered with a surfactant so as to prepare a medicine-containing complex; and the complex is solved or dispersed in an oil phase for making an external preparation. Above description can be referred to for specific conditions of implementation of the present invention method.

The present invention can improve the percutaneous absorbability of a medicine which has conventionally been difficult to use as an external preparation or which has not been able to offer sufficient effects as an external preparation because of low percutaneous absorbability.

In the following, examples and test examples are shown to explain the present invention in further detail; however, the scope of the present invention is not limited by the examples.

### EXAMPLES

### Production example 1: Production of S/O type complex suspension of the present invention

Into 10 mL of 10 mM phosphoric acid buffer solution (pH 8.0), diclofenac sodium (hereinafter referred to as "DFNa") at a concentration of 15 mg/mL and cow serum albumin (BSA, molecular weight: 69,000) at a concentration of 10 mg/mL were dissolved. To the solution, 20 mL of 5 wt% solution of sucrose erucic acid ester (manufactured by Mitsubishi-Kagaku Foods Corporation, ER-290, erucic acid 90 wt%, HLB: 2) in toluene was added. The mixture was stirred at high speed (26,000 rpm) by a homogenizer to prepare a W/O type emulsion. The emulsion was lyophiled for a day, to prepare a surfactant-DFNa complex. To this complex, 5 mL of soybean oil was added, followed by dispersion by irradiation of ultrasonic waves, to give an S/O type complex suspension of the present invention.

### Production example 2: Production of DFNa-S/O type ointment

To the DFNa-S/O type suspension prepared in Production example 1 at a concentration of 20 mass %, 10 mass % of macrogol, 5 mass % of diethyl sebacate, an appropriate amount of preservative, and petrolatum as residual were added, to prepare a DFNa-S/O type ointment.

### Production example 3: Production of DFNa-S/O type lotion agent

### A

DFNa-S/O type suspension of Production example 1 3.0 mass %

### B

| | |
|---|---|
| Tri-medium-chain fatty acid glyceride | 3.0 mass % |
| Sorbitan monostearate | 1.5 mass % |
| Acetic acid dl-α-tocopherol | 0.2 mass % |
| Preservative | appropriate amount |

### C

| | |
|---|---|
| 1,3-butyleneglycol | 7.0 mass % |
| Xanthan gum | 0.1 mass % |
| Purified water | residual |

After heating B at a temperature of 80°C for dissolution, A was gradually added to B and the mixture was stirred. Then C which had been dissolved homogenously was added, the mixture was stirred by a homo-mixer (5,000rpm) to be emulsified while keeping a temperature at 80°C. The mixture was cooled while being stirred with a paddle, stirring was stopped at a temperature of 40°C. The mixture was kept still to prepare a DFNa-S/O type lotion agent.

### Production example 4: Preparation of DFNa-S/O type aqueous cataplasm

| | |
|---|---|
| DFNa-S/O type suspension of Production example 1 | 5.0 mass % |
| Polyethylene glycol | 6.0 mass % |
| Sodium Polyacrylate | 6.0 mass % |
| Carmellose sodium | 6.0 mass % |
| Dihydroxyaluminium acetate | 0.1 mass % |
| Tartaric acid | 2.0 mass % |
| Glycerin | 20.0 mass% |
| Purified water | residual |

According to a conventional method, DFNa-S/O type aqueous cataplasms was prepared. Specifically, a DFNa-S/O type suspension prepared in Production example 1 was dissolved into polyethylene glycol, mixed with other materials until becoming homogenous, flatted directly on a polyester cloth. The cloth was cut in a desired size.

### Production example 5: DFNa-S/O type tape

| | |
|---|---|
| DFNa-S/O type suspension of Production example 1 | 5.0 mass % |
| Polyethylene glycol | 5.0 mass % |
| Styrene-isoprene-styrene block copolymer | 25. 0 mass % |
| Alicyclic saturated hydrocarbon resin | 45.0 mass % |
| Polybutene | 5.0 mass % |
| Liquid paraffin | 11.0 mass % |

A DFNa-S/O type aqueous tape preparation was prepared according to a conventional method. Specifically, styrene-isoprene-styrene block copolymer, alicyclic saturated hydrocarbon resin, polybutene, and liquid paraffin were heated and mixed at a temperature of 120 to 160°C. Then, DFNa-S/O type suspension prepared in Production example 1 dissolved in a polyethylene glycol was added and mixed. The mixture was flatted directly on a polyester cloth. The cloth was cut in a desired size.

### Production example 6: Production of S/O type complex suspension of the present invention

To a 10 mL of 10 mM phosphoric acid buffer solution (pH 8.0), a diclofenac sodium (hereinafter referred to as "DFNa") was dissolved at a concentration of 15 mg/mL. To the solution, 20 mL of 5 % by weight toluene solution of sucrose erucic acid ester (ER-290 manufactured by Mitsubishi-Kagaku Foods Corporation, erucic acid: 90% by weight, HLB: 2) was added. The mixture was stirred at high speed (26,000 rpm) by a homogenizer, to prepare a W/O type emulsion. The emulsion was lyophiled for a day, to prepare a surfactant-DFNa complex. To the complex, 5mL isopropyl myristate was added and ultrasound was applied to obtain an S/O type complex suspension according to the present invention.

### Production example 7: Production of S/O type complex suspension of the present invention

An S/O type complex suspension was prepared in a same manner to Production example 6 except that lecithin (manufactured by the Nisshin OilliO Group, Ltd.) instead of sucrose erucic acid ester and soybean oil instead of isopropyl myristate were used.

### Comparative production example 1: DFNa suspension

Before use, DFNa (0.75 g) was directly added to soybean oil (50 mL), and the mixture was subject to ultrasound and dispersed to prepare a control preparation.

### Test example 1: Percutaneous absorbability test

A percutaneous absorbability test was carried out by using ears of a New Zealand White male rabbit weighing about 6.0 Kg. A writing brush of about 10 mm width was soaked in a DFNa-S/O type suspension (15 mg/mL) prepared in Production example 1. By using the writhing brush, DFNa at a ratio of 5mg/kg per weight of the rabbit was applied inside the left ear in an area of approximately 50 mm × 50 mm. At 1, 2, 4, 6, 8 and 24 hours after the application, a blood sample was taken in an amount of about 2 mL from the ear vein of the outer side of the opposite ear, namely the right ear, to obtain blood plasma by centrifugation. Into the blood plasma, a phosphoric acid and a purified water were added, and the mixture was charged into an extraction column (OASIS MAX extraction cartridge manufactured by Nihon Waters K.K.) which had been activated with methanol and water beforehand. First, methanol was eluted for cleaning, then 5% formic acid in methanol was used as eluate to obtain an extract. The obtained extract was determined for a blood concentration of DFNa by an HPLC (column used: STR-ODS II Type S manufactured by SHINWA CHEMICAL INDUSTRIES, LTD.) in which 0.12% acetic acid: methanol = 3 : 5 is used as eluate.

Two weeks after the test described above, a soybean oil suspension (15 mg/mL) of DFNa prepared in the above Comparative production example 1 was administered orally with a sonde at half the above dose (DFNa of 2.5 mg/kg per weight of the rabbit). Then, blood concentration of DFNa was determined in a similar manner to the above.

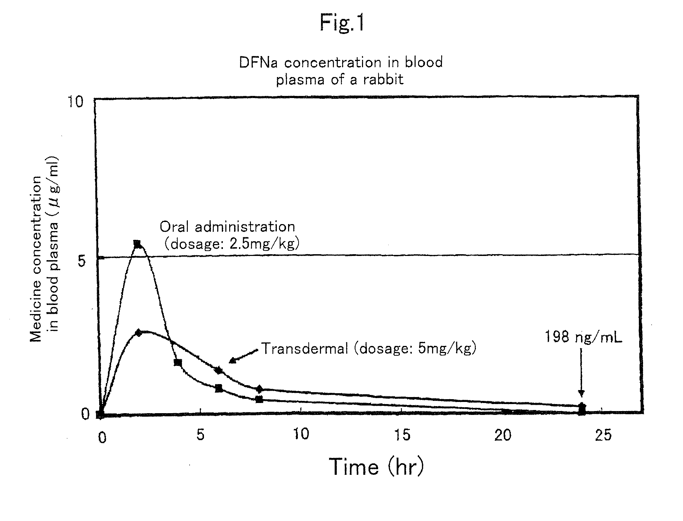

Fig. 1 shows a variation of blood concentration of DFNa when DFNa-S/O type suspension of Production example 1 was applied as a percutaneous absorption agent on the inner side of the left ear of a rabbit in an amount of 5mg per kg body weight, and, for comparison, when a soybean oil suspension of comparative production example 1 was administered orally in an amount of 2.5 mg per kg body weight using the same rabbit after drug holidays.

Tmax of the percutaneous pharmaceutical preparation showed an absorption speed equivalent to 2 hours which was almost same as that of oral preparation and which was more rapid than ever. The Cmax at that time was high at 2.5 µg/mL which was almost half that of the oral preparation. Additionally, the blood concentration of DFNa was high at 198 ng/mL even in the 24th hour, which demonstrated the excellent sustainability.

### Test example 2 Percutaneous absorbability test

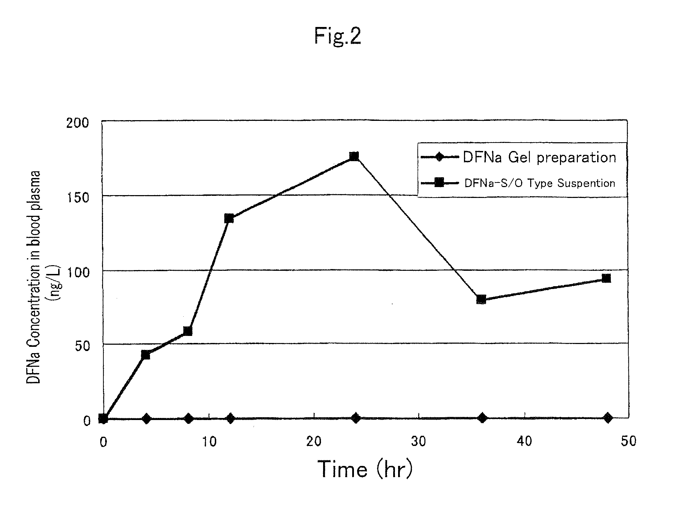

A large area of a back skin of each of two beagle dogs was clipped 3 days before the test by using a hair clipper in order not to damage the skin, and an area of 5 cm square was determined to be a region where preparation was to be applied. A specific amount of DFNa-S/O type suspension of production example 1 was placed in a syringe, and was applied once homogenously on the application region at a dose of 5 mg/kg. At a total of 7 points of time, i.e. after the administration and at 4, 8, 12, 24, 36, and 48 hours after the administration, a blood sample was taken by using a blood collecting tube containing heparin. The obtained blood was subjected to centrifugation to obtain blood plasma. The obtained blood plasma was determined for the blood concentration of DFNa in a similar manner to Test example 1. Additionally, a commercially available DFNa gel preparation (Voltaren Gel manufactured by Novartis Pharma) was applied homogenously, and blood concentration was determined in the same manner. The results are shown in Fig.2.

As shown in Fig.2, in the case of usual gel preparation, the blood concentration of DFNa was nearly at the detection limit or below. On the other hand, by applying the S/O type suspension of the present invention on the skin, blood concentration could be maintained for long periods of time without a sharp rise in blood concentration as in the case of oral preparation. Thus, it was demonstrated that the present invention is a highly excellent technique by which a medicine having low percutaneous absorbability could be made an external preparation.

### Test example 3: Safety test

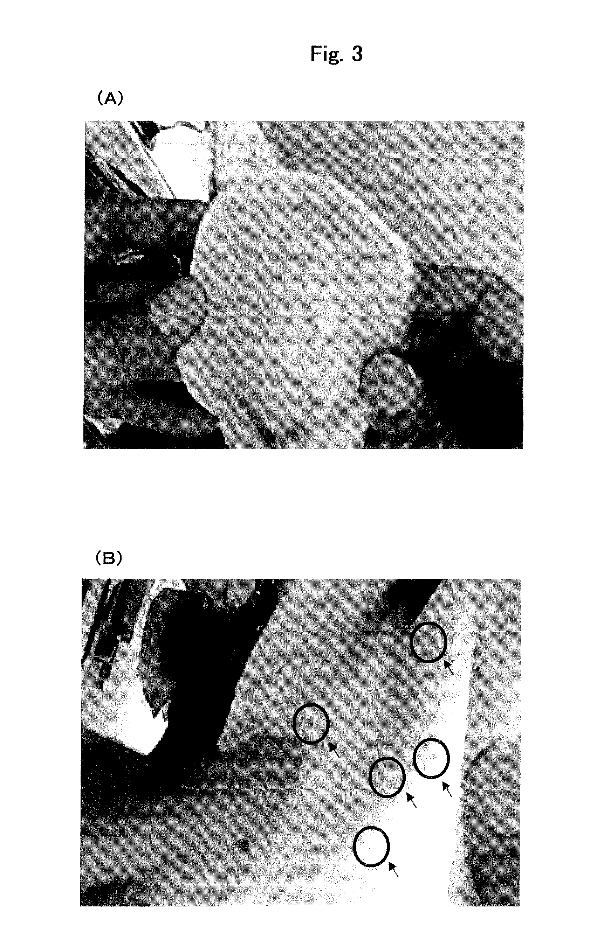

To the inner side of the left ear of a New Zealand White male rabbit weighing 6.0 Kg, the DFNa-S/O type suspension (15 mg/mL) prepared in Production example 1 was applied in the area of 50 mm × 50 mm in a similar manner to Test example 1. Additionally, a commercially available DFNa gel preparation (Voltaren Gel manufactured by Novartis Pharma) was applied almost in the equal dimention. Fig. 3 shows a photograph taken 24 hours after the application.

As in Fig. 3, when the conventional DNFa external preparation was applied, scar tissue, redness and the like developed. It is attributed to the fact that the preparation contains an isopropanol as a solvent. On the other hand, when the pharmaceutical preparation of the present invention was applied, no abnormality developed. Therefore, it was demonstrated that the pharmaceutical preparation of the present invention was highly safe.

### INDUSTRIAL APPLICABILITY

By the S/O type external preparation of the present invention, percutaneous absorbability of a hydrophilic medicine can be highly improved. Therefore, a medicine which has not conventionally been able to offer sufficient effects as an external preparation because of low percutaneous absorbability can be used as an external preparation. As a result, such a medicine can directly act in a diseased area while risks of side effect can be reduced. Thus, the S/O type external preparation and the method for improving percutaneous absorbability of a hydrophilic medicine according to the present invention have excellent industrial availability because the invention can extend the field of application of a hydrophilic medicine having a problem of low percutaneous absorbability.

## Claims

1. An S/O type external preparation excellent in percutaneous absorbability, comprising:
a medicine-containing complex dissolved or dispersed in an oil phase,
wherein the complex contains a hydrophilic medicine covered with a surfactant and is in a form of a solid.

2. The S/O type external preparation according to claim 1,
wherein the complex contains a protein and/or a polysaccharide as a stabilizing agent.

3. The S/O type external preparation according to claim 2,
wherein the protein and the polysaccharide have a molecular weight of 10,000 or more.

4. The S/O type external preparation according to any one of claims 1 to 3, wherein the surfactant is a nonionic surfactant.

5. The S/O type external pharmaceutical preparation according to any one of claims 1 to 4, wherein the surfactant is one or more than one selected from a group consisting of glycerin fatty acid ester, polyglycerin fatty acid ester, polyoxyethylene glycerin fatty acid ester, sorbitan fatty acid ester, sucrose fatty acid ester, polyoxyethylene sorbit fatty acid ester, polyoxyethylene castor oil, and hardened castor oil.

6. The S/O type external preparation according to any one of claims 1 to 5, wherein the surfactant has a HLB value of 10 or less.

7. The S/O type external preparation according to any one of claims 1 to 6, wherein the oil phase is one or more than one selected from a group consisting of soybean oil, sesame oil, olive oil, safflower oil, sunflower oil, rape seed oil, and perilla oil.

8. The S/O type external preparation according to any one of claims 1 to 6, wherein the oil phase is a neutral lipid or a long chain fatty acid ester.

9. The S/O type external preparation according to any one of claims 1 to 8, wherein the hydrophilic medicine is a non-steroidal anti-inflammatory drug.

10. The S/O type external preparation according to any one of claims 1 to 8, wherein the hydrophilic medicine is a diclofenac sodium.

11. A method for improving percutaneous absorbability of a hydrophilic medicine, comprising:
covering the hydrophilic medicine with a surfactant so as to prepare a medicine-containing complex, and
solving or dispersing the complex in an oil phase for making an external preparation.

12. The method according to claim 11, wherein a protein and/or a polysaccharide is added as a stabilizing agent to the complex.

13. The method according to claim 12, wherein the protein and the polysaccharide have a molecular weight of 10,000 or more.

14. The method according to any one of claims 11 to 13, wherein the surfactant is a nonionic surfactant.

15. The method according to any one of claims 11 to 14, wherein the surfactant is one or more than one selected from a group consisting of glycerin fatty acid ester, polyglycerin fatty acid ester, polyoxy ethylene glycerin fatty acid ester, sorbitan fatty acid ester, sucrose fatty acid ester, polyoxy ethylene sorbit fatty acid ester, polyoxy ethylene castor oil, and hydrogenated castor oil.

16. The method according to any one of claims 11 to 15, wherein the surfactant has a HLB value of 10 or less.

17. The method according to any one of claims 11 to 16, wherein the oil phase is one or more than one selected from a group consisting of soybean oil, sesame oil, olive oil, safflower oil, sunflower oil, rape seed oil, and perilla oil.

18. The method according to any one of claims 11 to 16, wherein the oil phase is a neutral lipid or a long chain fatty acid ester.

19. The method according to any one of claims 11 to 18, wherein the hydrophilic medicine is a non-steroidal anti-inflammatory drug.

20. The method according to any one of claims 11 to 18, wherein the hydrophilic medicine is a diclofenac sodium.
